(19) Europäisches Patentamt
European Patent Office
Office européen des brevets

(11) Numéro de publication: **0 039 265**
**B1**

(12) # FASCICULE DE BREVET EUROPEEN

(45) Date de publication du fascicule du brevet: **06.07.83**

(51) Int. Cl.³: **A 61 K 31/35**

(21) Numéro de dépôt: **81400550.0**

(22) Date de dépôt: **07.04.81**

(54) **Nouveau médicament à base de biflavanone.**

(30) Priorité: **22.04.80 FR 8008966**

(43) Date de publication de la demande:
**04.11.81 Bulletin 81/44**

(45) Mention de la délivrance du brevet:
**06.07.83 Bulletin 83/27**

(84) Etats contractants désignés:
**AT BE CH DE GB IT LI NL SE**

(56) Documents cités:
**CHEMICAL ABSTRACTS, vol. 83, 1975, page 97, ref. 142 488c Columbus, Ohio, US SUOLINNA et al.: »Effect of flavonoids on aerobic glycolysis and growth of tumar cells«**

(73) Titulaire: **SCIENCE UNION ET Cie SOCIETE FRANCAISE DE RECHERCHE MEDICALE, 14 rue du Val d'Or, F-92150 Suresnes (FR)**

(72) Inventeur: **Offner, Edouard, "Les Tilleuls" Vattetot sous Beaumont, F-76110 Goderville (FR)**
Inventeur: **Duhault, Jacques, 14 bis, rue Paul Demange, F-78290 Croissy S/ Seine (FR)**

(74) Mandataire: **Reverbori, Marcelle et al, SCIENCE UNION ET Cie SOCIETE FRANCAISE DE RECHERCHE MEDICALE Service Brevets 22 rue Garnier, F-92200 Neuilly/Seine (FR)**

**EP 0 039 265 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

La présente invention a pour objet un nouveau médicament pour le traitement des dysfonctionnements capillaires et veineux, et pour la prévention des complications du diabète, contenant comme principe actif une biflavanone de formule:

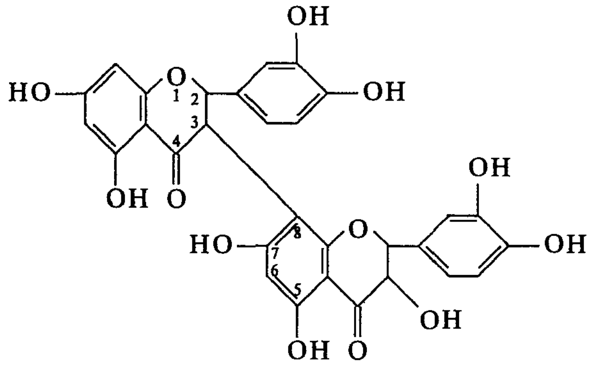

(I)

ou manniflavone.

Ce composé (I) est un produit connu [phytochemistry, 18, p. 1553 à 1557, (1979)], que l'on peut obtenir par extraction d'écorces deGARCINIA MANII qui est une espèce particulièrement abondante dans les forêts tropicales africaines. Un procédé d'extraction de ce produit est décrit dans l'article précité de Phytochemistry.

Parmi les nombreux composés extraits à partir de plantes de ce genre GARCINIA MANII, seuls les composés mono-flavonoides ont fait l'objet de nombreuses études qui révèlent une grande diversité dans leurs propriétés thérapeutiques.

Au cours d'essais pharmacologiques, nous avons découvert que, de façon surprenante, la biflavanone de formule (I) possédait une activité notable sur la résistance et la perméabilité des parois des vaisseaux sanguins et la propriété d'inhibition d'un enzyme, l'aldose réductase, propriété utile en particulier dans la prévention des complications de la maladie diabétique.

Le genre GARCINIA (Guttiferae) dont est issu le composé (I), est une source connue et abondante de biflavonoïdes (cf. Fortschr. Chem. Org. Naturst. [1973] 30, p. 207) et comprend plus de 200 espèces africaines et asiatiques, dont la particularité est de posséder deux restes flavonoïdiques liés par leurs carbones 3 et 8.

La biflavanone de l'invention peut être plus particulièrement isolée à partir des écorces de tiges de Garcinia Mannii avec des rendements supérieurs à 10% par rapport au poids sec des écorces.

Les méthodes d'isolement décrites jusqu'à present ne sont cependant pas très claires et ne conduisent pas à des composés d'une grande pureté. Or, nous avons trouvé que les extraits secs de composé biflavonique de formule I, isolés de façon classique à partir des écorces de tiges de Garcinia Mannii pouvaient être facilement purifiés par extraction avec une solution saturée de bicarbonate de sodium, reprise du composé par l'éther en milieu acide et précipitation dans l'hexane.

Les caractéristiques physiques du composé I sont les suivantes:

— Point de fusion:
  240—243° C

— Pouvoir rotatoire:
  $[\alpha]D^{22} = -20{,}3°$ (C = 1% dans l'acétone)

— Ultra-violet:
  $\lambda$ max. = 292 nm (épaulement à 335) (dans l'éthanol)

— Infra-Rouge:
  $\nu$ (OH) = 3600 à 3000 $cm^{-1}$
  $\nu$ (C=O) = 1630 $cm^{-1}$

— R.M.N. à 60 MHz (solvant acétone deutérié):
  $\delta$ = 5,75 et 4,8 ppm (2 H-AB-J = 12 Hz)
  attribuable aux protons 2 et 3 du 1er monomère
  $\delta$ = 5,1 et 4,4 ppm (2H-AB-J = 12 Hz)
  attribuable aux protons 2 et 3 du 2ème monomère
  $\delta$ = 7,2 et 6,8 ppm (6H aromatiques des cycles B).
  $\delta$ = 6,1 ppm (3H aromatiques des cycles A).

— Pureté:
Rf caractéristique en CCM : tâche unique à environ 0,35 dans un système $SiO_2$-toluéne-méthyl-éthylcétone-formiate d'éthyle-acide formique (4-1-4-1).

— Analyse:
malgré la présence de traces d'eau:
$C_{30}H_{22}O_{13}$
Calculé C 61,03, H 3,76,
Trouvé C 60,62, H 4,17.

Nous avons pu étudier les propriétés du composé biflavonique (I) ainsi purifié, et mettre en évidence ses intéressantes propriétés pharmacologiques jusqu'alors insoupçonnées pour ce type de composé.

## Propriétés pharmacologiques

### A) toxicité à haute dose

Elle a été déterminée sur des groupes homogènes de souris Swiss et NMRI mâles, de race pure, pesant de 19 à 22 g et sur des groupes homogènes de rats Sprague Dawley mâles adultes d'un poids moyen de 300 g.

En administration orale, la dose maximale toujours tolérée chez la souris Swiss comme chez la souris NMRI s'est montrée supérieure à 10 000 mg/kg.

En administration intrapéritonéale, aussi bien chez le rat que chez la souris, la dose maximale toujours tolérée est supérieure à 3 et 4000 mg/kg.

Nous pouvons donc dire que la biflavanone (I) est un composé absolument atoxique.

### B) activité pariétotrope

Elle a été recherchée sur deux espèces animales, le lapin et le rat.

#### 1. méthode de AMBROSE et DE EDS

Nous avons utilisé une méthode dérivée de celle décrite par AMBROSE et DE EDS [J. Pharm. Exp. Ther. 90, 359 (1947)]. Un lapin mâle fauve, d'un poids compris entre 2,0 et 2,5 kg, est épilé sur toute la région ventrale à l'aide d'une pâte au sulfure de baryum. Le lendemain l'animal reçoit, en injection intraveineuse 25 mg/kg d'une solution aqueuse de Bleu Trypan à 1 p. 100. Trente minutes après cette injection, on applique pendant 30 secondes un tampon de coton de 1,2 cm de côté, imbibé de chloroforme, en deux ou trois points de la zone épilée, et on note le tamps d'apparition de la couleur bleue.

Ce temps doit être sensiblement constant quel que soit le point d'application du tampon et traduit la plus ou moins grande rapidité avec laquelle le colorant quitte le courant sanguin pour imprégner les tissus sous-cutanés.

Le produit à tester est alors aussitôt administré soit par voie intra-péritonéale, soit par voie orale. L'agent irritant est appliqué de nouveau, à des intervalles de quinze minutes, en d'autres points de la zone épilée : on note à chaque fois le temps d'apparition de la coloration bleue.

On opère en principe sur des lots de sept lapins par dose et par produit. Il est à noter que:

- la réponse à l'agent irritant n'étant pas forcément identique selon le point d'application sur l'abdomen, il faut faire varier systématiquement »les points témoins« et les »points traités« d'un lapin à l'autre;
- il faut toujours considérer le temps d'apparition de la coloration bleue et non le temps de son développement complet;
- au bout d'une heure d'expérience, et à chaque heure ensuite, il faut refaire une injection intraveineuse de Bleu Trypan de 1 ml de solution aqueuse à 1 p. 100;
- chaque lapin est son propre témoin.

#### Résultats

Le protocole opératoire précédent a été utilisé et les doses suivantes ont été administrées : 500 mg et 1000 mg/kg. La biflavanone (I) a été ingérée sous forme de solution aqueuse additionnée de 5% de gomme adragante.

Les résultats sont évalués en pourcentage d'augmentation du temps d'apparition de la couleur bleue au niveau du point d'application par rapport au temps de base mesuré avant traitement, et comparés à des substances de référence administrées dans les mêmes conditions : catéchine hydrate, hespéridine, quercétine, rutine. Les résultats sont consignés dans le tableau ci-dessous, le maximum d'activité étant obtenu 6 à 7 heures environ après l'administration du produit.

| Voie orale | Augmentation % du temps d'apparition par rapport au temps témoin | | | | |
|---|---|---|---|---|---|
| Suspension Doses mg/kg | Biflavanone (I) | Catéchine hydrate | Hespéridine | Quercétine | Rutine |
| 500 | 50 | 38 | 0 | 0 | 27 |
| 1000 | 125 | 50 | 0 | 0 | 77 |

2. Méthode de BEACH et STEINETZ

La Technique d'AMBROSE et DE EDS a été modifiée afin de mesurer la coloration de la tache selon la technique de BEACH et STEINETZ [(J. Pharm. Expl. Therap. (1961) 131, n°1, p. 400 à 406). Des lapins New-Zealand mâles de 2,5 kg reçoivent une injection IP de produit à tester, ou d'eau gommée pour les témoins.

Trente minutes après, sur la peau abdominale préalablement épilée, un tampon de chloroforme est appliqué trente secondes, afin de créer une irritation locales qui favorise la diffusion du colorant hors des capillaires vers les liquides interstitiels.

Immédiatement après, une solution de Bleu Trypan à 25 mg/kg est injectée dans la veine marginale de l'oreille.

Une tache bleue apparait à l'endroit irrité. Le lapin est tué par injection intracardiaque de pentobarbital, 30 minutes après l'injection du Bleu Trypan.

Les fragments de peau sont prélevés, posés et laissés à digérer 24 heures dans HCl pur. Le Bleu Trypan est dosé selon la technique de BEACH et STEINETZ.

Après la dissolution complète, l'addition de 3 ml de chlorure de Benzalkonium en solution aqueuse à 12,80% complexe le colorant qui est ensuite repris par 5 ml de chloroforme. Après centrifugation, la phase chloroformique bleue est rapidement soutirée et filtrée, la lecture spectrophotométrique est faite immédiatement afin d'éviter une concentration du colorant par évaporation du chloroforme. Les densités optiques à 620 mn sont traduites en µg des bleu/100 mg de tissu.

Résultats

Le protocole opératoire précédent a été utilisé et les doses 200 et 400 mg ont été administrées. Les résultats, exprimés en µg de colorant par 100 g de peau fraiche sont donnés dans le tableau suivant:

| Concentration du bleu Trypan (µg/100 g de peau) | | | | |
|---|---|---|---|---|
| Solution I. P/kg (mg/kg) | Eau gommée | Biflavanone (I) | Diosmine | Rutine |
| 200 | 4,62 ± 1,4 | 4 ± 2,3 | 2,66 ± 2 | 3,6 ± 1,8 |
| 400 | 5,01 ± 2,3 | 3,1 ± 2,3 | 2,50 ± 1,6 | 3,2 ± 2 |

3. Autres méthodes

D'autres tests, utilisés chez le rat, ont confirmé les possibilités de la biflavanone dans la diminution de la perméabilité capillaire par rapport aux produits de référence classiques, et notamment les tests réalisés selon la technique de LEFEBVRE [(C.R. Soc. Biol. 1, 183 (1962)] qui consiste à observer l'apparition d'un colorant au niveau d'une injection intradermique d'histamine. Il a été constaté que l'infection intrapéritonéale de 100 mg/kg de biflavanone (I) retarde l'apparition de la coloration de 70%. La rutine à la même dose ne provoque qu'un retard de 12%.

Pour mettre en évidence l'action sur la résistance capillaire, nous avons utilisé la méthode décrite par J.L. PARROT et coll. [C.R. Soc. Biol. 140 750 (1946)]. Elle consiste à étudier les variations de la résistance capillaire chez le cobaye soumis à un régime spécial carencé en facteur vitaminique $P(C_2)$, mais équilibré en vitamine C. La résistance capillaire est mesurée au niveau de la région lombaire en appliquant sur la peau, préalablement épilée, une ventouse de verre de 5 mm de diamètre. On détermine la valeur de la dépression, exprimée en cm de mercure, qu'il faut réaliser pour faire apparaitre, au bout de 50 secondes environ, 3 pétéchies sur la surface entourée. Cette dépression est généralement comprise entre 25 et 35 cm de mercure, mais elle chute à 10 cm de Hg chez les animaux soumis au régime carencé pendant 10 jours.

On mesure ensuite la résistance capillaire chez les mêmes animaux, 3, 6, 8, 24 et 48 heures après administration des produits à tester. L'élévation de la résistance capillaire et sa durée sont exprimées planimétriquement par la valeur de la surface obtenue en portant sur un système de coordonnées les valeurs de la resistance capillaire en ordonnées et le temps en heures en abcisses.

On a constaté qu'en prenant la moyenne de 10 animaux, cette surface est de 20,8 $cm^2$ après injection de 50 mg/kg de biflavanone (I), tandis que la même dose de rutine ne donne que 6,42 $cm^2$.

Les effets pharmacologiques ci-dessus relatés permettent l'utilisation de ce composé, seul ou en association avec d'autres substances à activité vitaminique P, dans le traitement de nombreuses maladies et syndromes, provoqués par une fragilité vasculaire augmentée. Ainso on peut citer par ecemple les divers purpuras et diathèses hémorragiques, les ulcères gastroduidénaux, les colites ulcéreuses, les rétinopathies et les artériolites dans l'hypertension et le diabète, les métrorragies, les ulcères variqueux, et entre autre, la pathologie hépatique découlant d'une altération fonctionnelle des capillaires sinusoïdes.

C) Inhibition de l'aldose reductase

Le dérivé de formule générale I possède des propriétés phamacologiques intéressantes notamment des propriétés inhibitrices de l'enzyme aldose-réductase.

L'aldose-réductase est l'enzyme principal qui contrôle la régulation du métabolisme des aldoses et notamment des aldohexoses comme le galactose et le glucose en les transformant en polyol correspondant (sorbitol ou galactitol par exemple) dans l'organisme humain.

En présence d'un excès de glucose sanguin, l'activité d'un tel enzyme entraine une concentration anormale en galactitol ou en sorbitol chez les sujets galactosémiques ou hyperglycémiques. Des concentrations anormales en polyols entrainent l'accumulation de ces substances dans le cristallin, dans le système nerveux périphérique et dans les reins des sujets diabétiques. En effet, l'intervention de l'aldose-réductase présente dans le cristallin est peu sensible chez un sujet dont la glycémie est normale.

Son rôle devient beaucoup plus important chez les sujets diabétiques dont la glycémie est beaucoup plus élevée.

On explique ainsi une modification des fonctions capillaires et rénales, des troubles de la conduction nerveuse et l'apparition d'une cataracte diabétique avec perte de la transparence du cristallin. On vise donc à retarder à réduire ou éviter totalement l'apparition de ces complications graves du diabète par l'emploi d'un inhibiteur d'aldose-réductase.

L'activité inhibitrice d'aldose-réductase des composés de la présente invention a été mise en évidence par des essais in vitro sur l'aldose-réductase extraite de cristallins de rats selon la technique décrite par S. HAYMAN et J. H. KINOSHITA, J. Biol. Chem. 240 (1965) 877, modifiée par S. D. WARNA et J. H. KINOSHITA, Biochemical Pharmacology 5 (1976) 2505—2613.

La biflavanone (I), mise en suspension dans du tampon pH 7, est placée en incubation à 25° C dans un récipient bouché contenant l'aldose-réductase extrait de cristallins de rats CD River. Après 10 mn de contact, le substrat (aldéhyde d-glycérique) est introduit dans la cuve de réaction. La cinétique enzymatique est appréciée par la disparition, du milieu, du co-facteur Ac. Nicotinamide-adénine diphorphorique réduite (NADPH) selon la réaction:

$$D-\text{glucose} + \text{NADPH} + H^+ \rightarrow \text{Sorbitol} + \text{NADP}$$

On calcule l'activité enzymatique par détermination de la quantité de NADPH disparue. Les résultats sont exprimés en pourcentage de l'activité enzymatique de la préparation en l'absence de tout inhibiteur. Dans ces conditions on peut déterminer, par exemple, la dose minimale qui inhibe à 50% l'activité enzymatique.

L'inhibition à 50% de la préparation enzymatique est obtenue avec des concentrations s'échelonnant entre $10^{-7}$ M et 2,5 $10^{-7}$ M, et ce résultat se révèle être très supérieur à ceux obtenus non seulement avec les monoflavonoides usuels (e. a. diosmine), mais aussi les inhibiteurs d'aldose-réductase les plus récents (e. a. alrestatine):

Activité inhibitrice de l'aldose-réductase in vitro:

| Composé | inhibition à 50% |
|---|---|
| Biflavanone (I) | $10^{-7}$ à $2,5 \cdot 10^{-7}$ M |
| Diosmine | $10^{-4}$ à $5 \cdot 10^{-5}$ M |
| Alrestatine (Brevet US 38 21 383) | $10^{-6}$ M |

Les propriétés ci-dessus décrites dans le paragraphe C, jointes à la faible toxicité des composés de la présente invention, permettent leur utilisation en thérapeutique, notamment dans le traitment préventif et curatif des complikations dégéneratives du diabète, sont en particulier la cataracte, les angiopathies, les altérations des fonctions capillaires et rénales, et les troubles de la conduction nerveuse.

L'invention s'étend également aux compositions pharmaceutiques renfermant à titre de principe actif le composé de formule générale (I) en mélange ou en addition avec un excipient inerte non-toxique pharmaceutiquement acceptable, comme par exemple l'eau destillée, le glucose, le lactose, l'amidon, le talc, l'éthylcellulose, le stéarate de magnésium ou le beurre de cacao.

D'une manière préférée les composés de l'invention sont administrés par voie buccale, parentérale, rectale, locale ou sublinguale. Les formes pharmaceutiques qui conviennent plus particulièrement pour de telles administrations sont les solutions ou suspensions injectables conditionnées en ampoules, en flacons multidoses ou en seringues autoinjectables, les collyres, les comprimés nus ou entrobés, les dragées, les gélules, les pilules, les sirops ou les émulsions buvables, les comprimés sublinguaux et les suppositoires.

La posologie unitaire est variable selon la voie d'administration, l'âge du patient et la sévérité de l'indication thérapeutique. Elle peut s'échelonner entre environ 50 et environ 500 mg par prise unitaire. La posologie journalière peut s'échelonner entre environ 100 et environ 2000 mg chez l'adulte.

A titre d'exemples non limitatifs nous donnons ci-après des exemples de quelques compositions pharmaceutiques à base de biflavanone:

Exemple 1

| Préparation de gélules | pour une gélule |
|---|---|
| Biflavanone (I) | 0,250 g |
| talc | 0,005 g |
| dioxyde de silicium | 0,005 g |

On tamise les poudres, les mélange et répartit ensuite en gélules.

Exemple 2

| Préparation des comprimés | pour un comprimé |
|---|---|
| Biflavanone (I) | 0,100 g |
| stéarate de Magnésium | 0,050 g |
| amidon de maïs | 0,040 g |
| polyvidone | 0,030 g |

Le principe actif est mélangé avec l'amidon de maïs, mouillé avec une solution aqueuse de polyvidone, séché et calibré, puis lubrifié avec le stéarate. Le comprimé peut être éventuellement enrobé.

Exemple 3

| Préparation de sachets de granulés | pour un sachet |
|---|---|
| Biflavanone (I) | 0,100 g |
| lactose | 1 g |
| arôme citron en poudre | 0,025 g |
| arôme orange en poudre | 0,050 g |
| cyclamate de sodium | 0,050 g |
| acide citrique | 0,050 g |
| saccharose q. s. p. | 5 g |

On mélange les poudres après tamisage et les mouille avec de l'eau distillée. Le granulé est ensuite séché, calibré et réparti en sachets étanches.

Exemple 4

| Préparation d'une solution buvable | |
|---|---|
| Biflavanone (I) | 2 g |
| hydroxyéthylcellulose | 0,50 g |
| cyclohexylsulfamate de sodium | 0,25 g |
| paraoxybenzoate de méthyle | 0,08 g |
| paraoxybenzoate de propyle | 0,04 g |
| glycérine | 40 g |
| arôme de framboise | 5 ml |
| sirop de saccharose q. s. p. | 100 ml |

On prépare par dispersion et homogénéisation un gel fluide d'hydroxyéthylcellulose contenant les conservateurs (paraoxybenzoates). On ajoute le cyclohexylsulfamate, la glycérine, l'arôme et la biflavanone qui sont ensuite dispersés.

La préparation doit être agitée avant l'emploi. Une cuillère correspond ainsi à 0,250 g de principe actif.

Exemple 5

| Préparation de suppositoires | |
|---|---|
| Biflavanone (I) | 0,100 g |
| silice colloïdale | 0,100 g |
| glycérides semi-synthétiques q. s. p. | 3 g |

La préparation peut également contenir des agent antiinflammatoires, anesthésiques, antiseptiques.

**Revendications**

1. médicament contenant une biflavanone de formule:

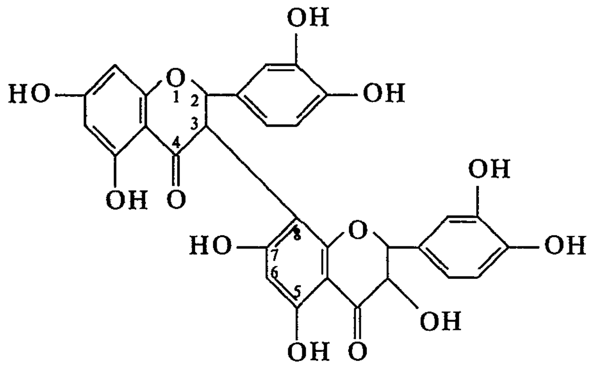

(I)

utilisable dans le traitement des maladies, résultant des troubles de la perméabilité et de la fragilité des parois vasculaires et des atteintes artérielles, veineuses ou capillaires, ainsi que dans la prévention de complications du diabète.

2. Les compositions pharmaceutiques contenant comme principe actif la biflavanone (I), seul ou en association avec d'autres substances à activité vitaminique P et des supports pharmaceutiques appropriés.

3. Les compositions pharmaceutiques, selon la revendication 2, présentées sous une forme convenant pour l'administration orale, rectale, parentérale ou percutanée, dans le traitement de diverses manifestations de la fragilité des parois vasculaires et des complications du diabète, seul ou associé à d'autres substances à activité complémentaire.

**Patentansprüche**

1. Medikament, enthaltend ein Biflavanon mit der allgemeinen Formel:

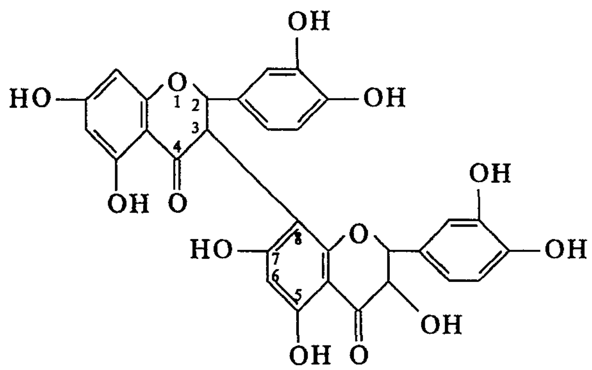

(I)

zur Verwendung bei der Behandlung von Krankheiten, die durch Störungen der Permeabilität und durch Brüchigkeit der Gefäßwände und durch arterielle, venöse oder kapilläre Schäden verursacht werden sowie zur Vorbeugung der Komplikationen der Diabetes.

2. Pharmazeutische Zusammensetzung enthaltend als wirksamen Bestandteil das Biflavanon (I), allein oder in Verbindung mit anderen Substanzen mit Vitamin P Aktivität und geeignete pharmazeutische Trägersubstanzen.

3. Pharmazeutische Zusammensetzung nach Anspruch 2, vorliegend in einer Form, die geeignet ist zur oralen, rektalen, parenteralen oder perkutanen Verabreichung bei der Behandlung der verschiedenen Manifestationen der Brüchigkeit der Gefäßwände und der Komplikationen der Diabetes, allein oder in Verbindung mit anderen Substanzen mit komplementärer Aktivität.

**Claims**

1. Medicament containing a biflavanone of the formula

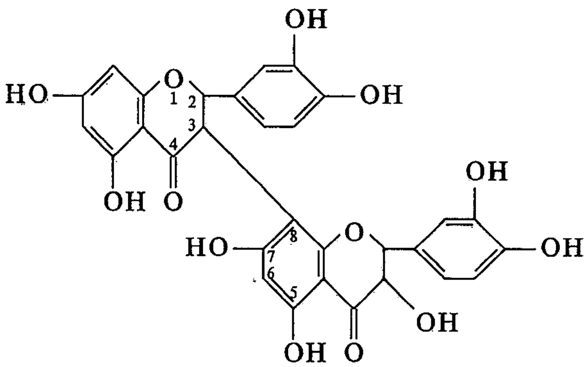

(I)

which can be used in the treatment of illnesses resulting from disorders in the permeability and the fragility of the vascular walls and arterial, venous or capillary damage, as well as in the prevention of complications in diabetes.

2. Pharmaceutical compositions containing, as the active ingredient, biflavanone (I), alone or together with other substances having a vitamin P acitivity, and suitable pharmaceutical carriers.

3. Pharmaceutical compositions according to claim 2, in a form suitable for oral, rectal, parenteral or percutaneous administration, in the treatment of various form of vascular-wall fragility and complications in diabetes, alone or together with other substances having complementary activity.